(19) Europäisches Patentamt
European Patent Office
Office européen des brevets

(11) EP 1 942 123 A1

(12) EUROPEAN PATENT APPLICATION
published in accordance with Art. 153(4) EPC

(43) Date of publication:
**09.07.2008 Bulletin 2008/28**

(21) Application number: **06810823.2**

(22) Date of filing: **29.09.2006**

(51) Int Cl.:
***C08G 18/32*** *(2006.01)* ***C07H 3/06*** *(2006.01)*

(86) International application number:
**PCT/JP2006/319417**

(87) International publication number:
**WO 2007/040163 (12.04.2007 Gazette 2007/15)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.10.2005 JP 2005291818**
**04.10.2005 JP 2005291819**

(71) Applicants:
- **Kaneka Corporation**
**Kita-ku**
**Osaka-shi, Osaka 530-8288 (JP)**
- **KABUSHIKI KAISHA HAYASHIBARA SEIBUTSU KAGAKU KENKYUJO**
**Okayama-shi, Okayama 700-0907 (JP)**

(72) Inventors:
- **AWAJI, Hiroshi**
**Settsu-shi, Osaka 566-0072 (JP)**
- **KUMAR, Ashutosh**
**Settsu-shi, Osaka 566-0072 (JP)**

(74) Representative: **HOFFMANN EITLE**
**Patent- und Rechtsanwälte**
**Arabellastrasse 4**
**81925 München (DE)**

# (54) POLYURETHANE DERIVATIVE, POLYURETHANE FOAM, AND PROCESS FOR PRODUCING THEM

(57) This invention provides a novel optionally acylated cyclic tetrasaccharide-containing polyurethane derivative and a process for producing the same, and a novel cyclic tetrasaccharide modified polyurethane foam and a simple process for producing the same. Specifically, the present invention provides a polyurethane derivative containing an optionally acylated cyclic tetrasaccharide: cyclo{→6)-α-D-glucopyranosyl-(1→3)-α-D-glucopyranosyl-(1 →6)-α-D-glucopyranosyl-(1→3 )-α-D-glucopyranosyl-(1→3}, a process for producing polyurethane, comprising reacting an acylated cyclic tetrasaccharide and a diol compound with a diisocyanete compound, a process for producing a cyclic tetrasaccharide-containing polyurethane, comprising hydrolyzing the polyurethane, a process for producing a polyurethane foam, comprising reacting polyol with polyisocyanete in the presence of the cyclic tetrasaccharide and foaming the reaction product, and a cyclic tetrasaccharide modified polyurethane foam produced by the production process.

EP 1 942 123 A1

## Description

Technical Field

The present invention relates to an optionally acylated cyclic tetrasaccharide-containing polyurethane derivative, a process for producing an acylated cyclic tetrasaccharide-containing polyurethane by reacting an acylated cyclic tetrasaccharide and a diol compound with a diisocyanate compound, a process for producing a cyclic tetrasaccharide-containing polyurethane by hydrolyzing the same, and a process for producing a cyclic tetrasaccharide-containing polyurethane by reacting a cyclic tetrasaccharide and a diol compound with a diisocyanate compound. The present invention also relates to a cyclic tetrasaccharide-containing polyurethane foam excellent in water-absorbing property, obtainable by reaction of a cyclic tetrasaccharide, a polyol other than the cyclic tetrasaccharide, and a diisocyanate, as well as a process for producing the polyurethane foam.

Background Art

Polyurethane is a polymer formed by addition polymerization of basically two kinds of main raw materials, that is, a polyol and a diisocyanate. The polyurethane is used in a cushion material, a heat insulating material, a sealing material, a waterproofing material, a floor material, a paver material, a coating material, an adhesive, an artificial leather, elastic fibers, a member for sports gear, a bandage, a cast, a catheter, and the like, in a wide range of fields such as automobiles, electronic products, civil engineering and construction, household goods, and medical care.

Recently, there has been developed a polyurethane endowed not only with water-absorbing property, antithrombogenicity, or the like, as high-performance properties of a polyurethane, but also with biodegradability containing biomass materials that are saccharides such as monosaccharide, disaccharide, oligosaccharide and polysaccharide in order to reduce influence on global environment by reduction in use of fossil resources. For example, there are disclosed a polyurethane containing cyclodextrin as a polyurethane containing a cyclic oligosaccharide (see, for example, Patent Documents 1 and 3), a polyurethane containing starch and its variant molasses or a polysaccharide (see, for example, Patent Documents 2 and 5), a polyurethane containing a monosaccharide, a disaccharide, an oligosaccharide or a polysaccharide (see, for example, Patent Document 4), and a polyurethane containing, in its side chain, an acylated monosaccharide, disaccharide, oligosaccharide or polysaccharide, and its deacylated polyurethane (see, for example, Patent Document 6).

A polyurethane foam is low in density and is characteristic in its resilient behavior, and is thus used widely in vehicles, furniture, bedding members, medical members, or the like. For production of the polyurethane foam, a one-shot method is generally used wherein at least four components of a first component containing a polyol, a foam stabilizer, and the like, a second component containing a catalyst such as a tin-based catalyst, a third component containing a polyisocyanate, and water are mixed and then simultaneously subjected to a reaction including a chain extension reaction and a crosslinking reaction and making a foam with carbon dioxide generated upon decomposition of the polyisocyanate with water,thereby forming a foam.

Recently, it is attempted to develop a polyurethane which contains biomass materials that are saccharides such as monosaccharide, disaccharide, oligosaccharide and polysaccharide which is endowed with water-absorbing property and biodegradability for the purpose of high-performance properties of a polyurethane, and also in order to reduce influence on global environment by reduction in use of fossil resources.

For example, there are disclosed a polyurethane foam containing cyclodextrin that is a cyclic oligosaccharide (see, for example, Patent Document 3) and a polyurethane foam containing starch and its variant, molasses or a polysaccharide (see, for example, Patent Document 5).

On the one hand, various derivatives of one kind of cyclic tetrasaccharide that is the minimum natural-derived cyclic oligosaccharide known at present, that is, cyclo{→6)-α-D-glucopyranosyl-(1→3)-α-D-glucopyranosyl-(1 →6)-α-D-glucopyranosyl-(1→3)-α-D-glucopyranosyl- (1→} (see the following formula) are known (see, for example, Patent Document 7).

[Chemical formula 1]

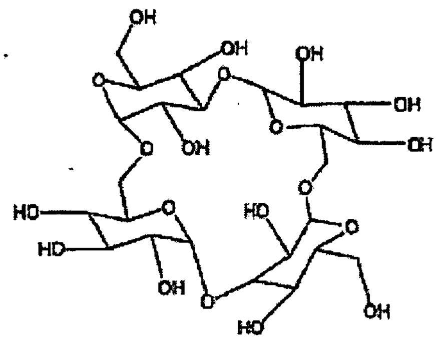

However, a cyclic tetrasaccharide-containing polyurethane and polyurethane foam and a process for producing the same are not known at all.
Patent Document 1: JP-A 5-86103
Patent Document 2: JP-A 5-186556
Patent Document 3: JP-A 7-53658
Patent Document 4: JP-A 9-12588
Patent Document 5: JP-A 9-104737
Patent Document 6: JP-A 11-71391
Patent Document 7: JP-A 2003-160595

Disclosure of Invention

Problem to be solved by the Invention

An object of the present invention is to provide an optionally acylated cyclic tetrasaccharide-containing novel polyurethane derivative and a process for producing the same. Another object of the present invention is to provide a cyclic tetrasaccharide-modified polyurethane foam as a novel polyurethane foam excellent in water-absorbing property, as well as a process for easily producing the polyurethane foam.

Means for solving the Problem

The inventors made extensive study in light of the problem described above, and as a result, the inventors found that an acylated cyclic tetrasaccharide having two primary hydroxyl groups is subjected to addition polymerization with a diol compound and a diisocyanate, thereby giving a novel polyurethane derivative containing an acylated cyclic tetrasaccharide, and also that the acylated cyclic tetrasaccharide-containing polyurethane is deacylated, or a cyclic tetrasaccharide is reacted with a polyol and a diisocyanate, thereby giving a novel polyurethane derivative containing a cyclic tetrasaccharide, and on the basis of these findings, or the like, the present invention was completed.
That is, the present invention relates to the following (1) to (10):

(1) An acylated cyclic tetrasaccharide-containing polyurethane represented by the following general formula (1):

[Formula 1]

$$-\{[CO-NH-R^1-NH-CO]-[O-R^2-O]\}m-\#$$

$$\#-\{[CO-NH-R^1-NH-CO]-[O-\underset{(OH)_{10-p}}{\overset{(OR^3)_p}{CTS}}-O]\}n- \qquad [1]$$

wherein $R^1$ represents a divalent aliphatic hydrocarbon group having 4 to 16 carbon atoms, a divalent aromatic hydrocarbon group having 6 to 16 carbon atoms, or a divalent aromatic substituted hydrocarbon group having 7 to 16 carbon atoms, $R^2$ represents a divalent organic group containing 1 to 100 units in total of an oxyalkylene group having 2 to 12 carbon atoms and/or an alkylene group having 2 to 6 carbon atoms and when there are a plurality of oxyalkylene groups and a plurality of alkylene groups, they may be the same or different, respectively, $R^3$ represents an acyl group having 2 to 8 carbon atoms, CTS represents a skeleton of a cyclic tetrasaccharide that is cyclo{→6)-α-D-glucopyranosyl-(1→3)-α-D-glucopyranosyl-(1 →6)-α-D-glucopyranosyl-(1→3)-α-D-glucopyranosyl-(1→}, represented by the following formula:

[Chemical formula 2]

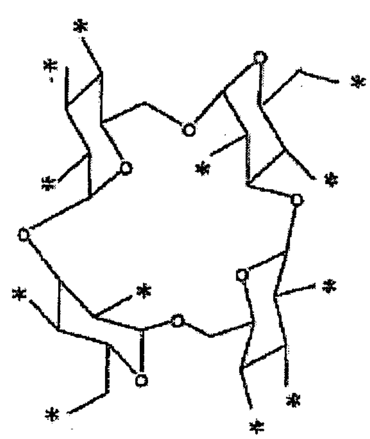

wherein * represents the binding position of a hydroxyl group.

In the formula (1), p represents an integer of 1 to 10, m and n each represent the number of repeating units, m is an integer of 0 to 1000, n is an integer of 1 to 1000, and n/(m + n) is a number in the range of 0.01 to 1; when there are a plurality of $R^1$s, $R^2$s or $R^3$s, each of $R^1$s, $R^2$s or $R^3$s may be the same or different, and when there are a plurality of CTS, the positions of CTS to which $R^3$ is introduced may be the same or different.

(2) The above-mentioned acylated cyclic tetrasaccharide-containing polyurethane wherein $R^3$ is an acetyl group.

(3) A cyclic tetrasaccharide-containing polyurethane represented by the following general formula (2):

[Formula 2]

$$-\{[CO-NH-R^1-NH-CO]-[O-R^2-O]\}m-\#$$

$$\#-\{[CO-NH-R^1-NH-CO]-[O-\underset{\displaystyle (OH)_{10}}{\underset{|}{CTS}}-O]\}n- \qquad [2]$$

wherein $R^1$ represents a divalent aliphatic hydrocarbon group having 4 to 16 carbon atoms, a divalent aromatic hydrocarbon group having 6 to 16 carbon atoms, or a divalent aromatic substituted hydrocarbon group having 7 to 16 carbon atoms, $R^2$ represents a divalent organic group containing 1 to 100 units in total of an oxyalkylene group having 2 to 12 carbon atoms and/or an alkylene group having 2 to 6 carbon atoms and when there are a plurality of oxyalkylene groups and a plurality of alkylene groups, they may be the same or different, respectively, CTS has the same meaning as defined above, m and n each represent the number of repeating units, m is an integer of 0 to 1000, n is an integer of 1 to 1000, and n/(m + n) is a number in the range of 0.01 to 1; and when there are a plurality of $R^1$s or $R^2$s, each of $R^1$s or $R^2$s may be the same or different.

(4) A process for producing an acylated cyclic tetrasaccharide-containing polyurethane represented by the general formula (1), comprising
reacting an acylated cyclic tetrasaccharide represented by the following general formula (3):

[Formula 3]

$$HO-\overset{\displaystyle (OR^3)_p}{\overset{|}{\underset{\displaystyle (OH)_{10-p}}{\underset{|}{CTS}}}}-OH \qquad [3]$$

wherein $R^3$ represents an acyl group having 2 to 8 carbon atoms, p represents an integer of 1 to 10, CTS has the same meaning as defined above, and when there are a plurality of $R^3$s, $R^3$s may be the same or different, and
a diol represented by the following general formula (4) :

[Formula 4] **$HO-R^2-OH$** **[4]**

wherein $R^2$ represents a divalent organic group containing 1 to 100 units in total of an oxyalkylene group having 2 to 12 carbon atoms and/or an alkylene group having 2 to 6 carbon atoms and when there are a plurality of oxyalkylene groups and a plurality of alkylene groups, they may be the same or different, respectively, with
a diisocyanate represented by the following general formula (5):

[Formula 5] $O=C=N-R^1-N=C=O$ [5]

wherein $R^1$ represents a divalent aliphatic hydrocarbon group having 4 to 16 carbon atoms, a divalent aromatic hydrocarbon group having 6 to 16 carbon atoms, or a divalent aromatic substituted hydrocarbon group having 7 to 16 carbon atoms.

(5) A process for producing the cyclic tetrasaccharide-containing polyurethane represented by the general formula (2), comprising hydrolyzing an acyl-group moiety of the acylated cyclic tetrasaccharide represented by the general formula (1).

(6) A process for producing the cyclic tetrasaccharide-containing polyurethane represented by the general formula (2), comprising
reacting a cyclic tetrasaccharide represented by the following general formula (6):

[Formula 6]

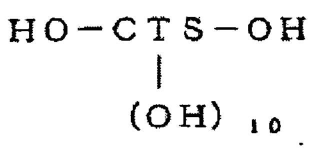

[6]

wherein CTS has the same meaning as defined above, and a diol represented by the general formula (4), with a diisocyanate represented by the general formula (5).

(7) A polyurethane foam comprising a cyclic tetrasaccharide that is cyclo{→6)-α-D-glucopyranosyl-(1→3)-α-D-glucopyranosyl-(1 →6)-α-D-glucopyranosyl-(1→3)-α-D-glucopyranosyl-(1→} (hereinafter referred to simply as cyclic tetrasaccharide), a polyol other than the cyclic tetrasaccharide (hereinafter referred to simply as polyol), and a polyisocyanate.

(8) A process for producing a polyurethane foam having a cyclic tetrasaccharide, comprising reacting a polyol other than a cyclic tetrasaccharide with a polyisocyanate in the presence of a cyclic tetrasaccharide that is cyclo{→6)-α-D-glucopyranosyl-(1→3)-α-D-glucopyranosyl-(1 →6)-α-D-glucopyranosyl-(1→3)-α-D-glucopyranosyl-(1→), and simultaneously making a foam by using, as a part or the whole of a foaming agent, water in an amount of 1 to 5 parts by weight based on 100 parts by weight of the polyol and the cyclic tetrasaccharide in total.

(9) The process for producing a polyurethane foam according to the above-mentioned (8), wherein the polyol has 2 to 4 functional groups on average and a hydroxyl value of 30 to 240 mg KOH/g.

(10) The process for producing a polyurethane foam according to the above-mentioned (8) or (9), wherein the polyisocyanate is tolylene diisocyanate.

## Effect of the Invention

The acylated cyclic tetrasaccharide-containing polyurethane of the present invention is thermoplastic and excellent in film formability. The cyclic tetrasaccharide-containing polyurethane of the present invention is thermoplastic and excellent in film formability and water-absorbing property. Accordingly, these polyurethanes are useful as highly functional materials in the field of household goods, or the like.

The polyurethane foam of the present invention is a polyurethane foam having a novel structure having a cyclic tetrasaccharide. With respect to the process for producing a polyurethane foam according to the present invention, a polyurethane foam with improved water-absorbing property without substantially changing or deteriorating other physical properties can be obtained by reacting a polyol with a polyisocyanate in the presence of a cyclic tetrasaccharide, if necessary together with a catalyst, a foam stabilizer and a foaming agent. The polyurethane foam having a cyclic tetrasaccharide according to the present invention can be used widely in vehicles, furniture, bedding members, or the like.

## Best Mode for Carrying Out the Invention

In the general formulae (1), (2) and (5), $R^1$ is a divalent aliphatic hydrocarbon group having 4 to 16 carbon atoms, a divalent aromatic hydrocarbon group having 6 to 16 carbon atoms, or a divalent aromatic substituted hydrocarbon group having 7 to 16 carbon atoms. When these groups have a chain structure, the chain may be linear or branched. Specific examples of the $R^1$ group include, for example, divalent groups such as a tetramethylene group, a pentamethylene group, a hexamethylene group, an octamethylene group, a hexadecamethylene group, a vinylene group, a propenylene group, a phenylene group and a naphthylene group, as well as a divalent hydrocarbon group derived from a monovalent hydrocarbon group (for example, a methylphenyl group, an ethylphenyl group, a biphenyl group, a methylene bisphenyl group, an ethylene bisphenyl group or the like) by removing a hydrogen atom bound to an aromatic ring thereof. Among these groups, a methylene bisphenyl group, a methylphenyl group and a hexamethylene group are preferable.

In the general formulae (1), (2) and (4), $R^2$ is a divalent organic group containing 1 to 100 units in total of an oxyalkylene group having 2 to 12 carbon atoms and/or an alkylene group having 2 to 6 carbon atoms and when there are a plurality of oxyalkylene groups and a plurality of alkylene groups, they may be the same or different, respectively. Such a group may be a divalent organic group containing 1 to 100 units in total which are the same or different and each represent an oxyalkylene group having 2 to 12 carbon atoms, a divalent organic group containing 1 to 100 units in total which are the same or different and each represent an alkylene group having 2 to 6 carbon atoms, or a divalent

organic group containing 2 to 100 units in total of an oxyalkylene group having 2 to 12 carbon atoms and an alkylene group having 2 to 6 carbon atoms and when there are a plurality of oxyalkylene groups and a plurality of alkylene groups, they may be the same or different, respectively. Specifically, $R^2$ is for example (1) a $-(BO)_{h-1}-B-$ group, wherein B represents an alkylene group having 2 to 12 carbon atoms, h represents a number of 1 to 100 indicative of the average number of moles of oxyalkylene groups added, and when there are a plurality of B' s, B' s may be the same or different. The repeating unit (BO group) may be linear or branched. Specific examples of such a repeating unit (BO group) include, for example, alkyleneoxy groups such as an ethyleneoxy group, a propyleneoxy group, a trimethyleneoxy group, a butyleneoxy group and a tetramethyleneoxy group. $R^2$ may be for example (2) a group having repeating units including alkylene ester groups such as an ethylene adipate group, a propylene adipate group, a butylene adipate group, a hexamethylene adipate group and a neopentyl adipate group, alkylene carbonate groups such as a hexamethylene carbonate group, and a ring-opened caprolactone group (specifically, a divalent group derived frompolyethylene adipate diol or the like by removing OH groups at both ends thereof, or the like). $R^2$ may also be for example (3) a$-(E)_i-$group, wherein E represents a divalent hydrocarbon group having 2 to 6 carbon atoms, and i represents a number of 1 to 100 indicative of the average number of moles of E units added, and when there are a plurality of E' s, E' s may be the same or different. The repeating unit represented by E may be linear or branched or may be a saturated group or an unsaturated group. A hydrogen atom on E may be replaced by another atom or substituent. Specific examples of such a repeating unit include divalent groups such as an ethylene group, a trimethylene group, a tetramethylene group, a hexamethylene group, a nonamethylene group, a $-CH_2-CF_2-CF_2-CF_2-CF_2-CH_2-$ group, a butadienylene group, a hydrogenated butadienylene group, a group derived from hydrogenated isoprene by removing one hydrogen atom from each of carbon atoms at both ends thereof, and a polydimethylsiloxydimethylsilyl-n-propylbisethoxy group, and the like. Preferable examples of $R^2$ among these groups include an ethyleneoxy group, a propyleneoxy group, an ethylene adipate group, a propylene adipate group, a hexamethylene carbonate group, a ring-opened caprolactone group, a trimethylene group, a tetramethylene group, a $-CH_2-CF_2-CF_2-CF_2-CF_2-CH_2-$ group, a hydrogenated butadienylene group, a group derived from hydrogenated isoprene by removing one hydrogen atom from each of carbon atoms at both ends thereof, and a polydimethylsiloxydimethylsilyl-n-propylbisethoxy group.

In the formulae (1) and (3), $R^3$ is an acyl group having 2 to 8 carbon atoms. Specific examples of $R^3$ include, for example, an acetyl group, a propionyl group, a butyryl group, an isobutyryl group, a valeryl group, an isovaleryl group, a pivaloyl group, a hexanoyl group, an octanoyl group, and the like. Among these groups, an acetyl group is preferable.

In the general formulae (1), (2), (3) and (6), CTS represents a skeleton of the cyclic tetrasaccharide shown above, and * represents the binding position of a hydroxyl group. When there are a plurality of CTSs, the positions of CTS to which $R^3$ is introduced may be the same or different.

In the formulae, p represents an integer of 1 to 10, preferably 5 to 10, from the viewpoint of water-absorbing property, m and n each represent the number of repeating units, m is 0 to 1000, preferably 10 to 1000, from the viewpoint of polymer strength and polymer formability, n is 1 to 1000, preferably 10 to 1000, from the viewpoint of water-absorbing property, and $n/(m + n)$ is a number in the range of 0.01 to 1, preferably 0.02 to 0.80, from the viewpoint of the balance among water-absorbing property, polymer strength and polymer formability. The repeating units in the general formulae (1) and (2) may be arranged regularly or at random.

Then, a process for producing the acylated cyclic tetrasaccharide-containing polyurethane represented by the general formula (1) is described by reference to the following (a) and (b):

(a) Structure of acylated cyclic tetrasaccharide:

A process for producing various cyclic tetrasaccharide derivatives is outlined in JP-A 2003-160595, and more specifically the acylated cyclic tetrasaccharide represented by the general formula (1) can be produced for example by the following method.

In the cyclic tetrasaccharide represented by the general formula (6), there are two primary hydroxyl groups as shown below:

[Chemical formula 3]

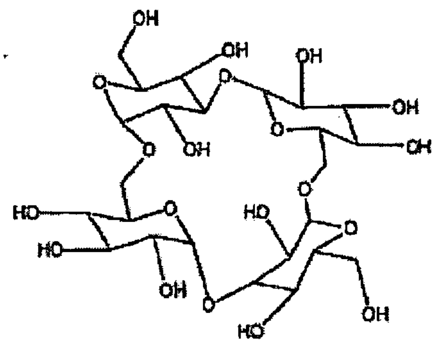

The two primary hydroxyl groups only are silylated with a silylating agent. The silylating agent is preferably t-butyldimethylsilyl chloride, t-butyldiphenylsilyl chloride, or triisopropylsilyl chloride that reacts more rapidly with a primary hydroxyl group than with a secondary hydroxyl group.

The amount of the silylating agent used is 2 to 2.2 moles, preferably 2.05 to 2.1 moles, per mole of the cyclic tetrasaccharide.

In the silylation, a catalyst is preferably used. As the catalyst, 4-dimethylaminopyridine or imidazole, for example, can be used in an amount of 5 to 15 mol%, preferably 7 to 10 mol%, based on the cyclic tetrasaccharide.

As the solvent used in the silylation reaction, a solvent in which a cyclic tetrasaccharide is dissolved, such as pyridine, N-methyl pyrrolidone or dimethyl formamide is used. Pyridine easily removable by distillation is most preferable.

The reaction temperature and time are 0 to 5°C and 1 to 2 hours at the time of adding a silylating agent and are 10 to 30°C and 1 to 2 hours thereafter.

After the reaction, the solvent is removed as much as possible by distillation, and then water that is 2 to 5 times as much as the solvent used is added to the reaction mixture and then stirred thereby precipitating a silylated cyclic tetrasaccharide. This product is filtered, washed with a sufficient amount of water and vacuum-dried to give a silylated cyclic tetrasaccharide represented by the following general formula (7):

[Formula 7]

$$R^4{}_3SiO^*-\underset{\displaystyle (OH)_{10}}{\underset{|}{CTS}}-O^*SiR^4{}_3 \ 7] \qquad [7]$$

wherein $R^4{}_3Si$ represents a t-butyldimethylsilyl group, a t-butyldiphenylsilyl group, or a triisopropylsilyl group, O* is an oxygen atom derived from a primary hydroxyl group, and CTS has the same meaning as defined above. Then, this silylated cyclic tetrasaccharide is reacted with an excessive amount of an acylating agent, to acylate all or some of the remaining secondary hydroxyl groups, thereby synthesizing an acylated silylated cyclic tetrasaccharide represented by the following general formula (8):

[Formula 8]

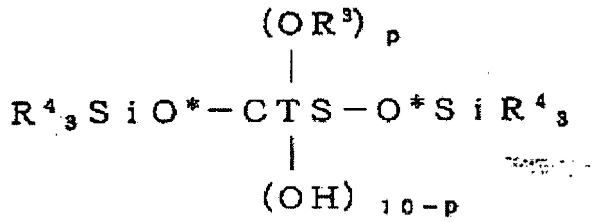 [8]

wherein $R^3$, $R^4{}_3Si$, CTS, p, and O* have the same meanings as defined above.

The acylating agent used is an acylating agent having 2 to 8 carbon atoms, for example, an acid anhydride having 2 to 8 carbon atoms (for example, acetic anhydride, propionic anhydride), an acid halide (for example, acetyl chloride, benzoyl chloride) or the like. The acylating agent is preferably an acetylating agent having 2 carbon atoms, most preferably acetic anhydride. The amount of the acylating agent used is 30 to 70 moles, preferably 40 to 60 moles, per mole of the silylated cyclic tetrasaccharide.

As the catalyst, 4-dimethylamiopyridine or imidazole is used preferably in an amount of 5 to 15 mol%, more preferably 10 mol%, based on the cyclic tetrasaccharide.

The reaction temperature is 60 to 90˚C, preferably 70 to 80˚C, and the reaction time is 10 to 24 hours, preferably 15 to 20 hours.

When the acylated silylated cyclic tetrasaccharide is purified, a preferable purification method is for example a method wherein the reaction mixture is concentrated and then introduced into water to form precipitates which are then filtered, washed with water and dried followed by chromatographic separation or recrystallization. In this case, the acylated silylated cyclic tetrasaccharide may be a single substance or a mixture of acylated silylated cyclic tetrasaccharides different in the degree of substitution of the acyl group.

Then, the acylated silylated cyclic tetrasaccharide is reacted with a desilylating agent thereby converting it into an acylated cyclic tetrasaccharide represented by the general formula (3). Preferable examples of the desilylating agent include tetra-n-butyl ammonium fluoride, tetraethyl ammonium fluoride, and tetramethyl ammonium fluoride. The amount of the desilylating agent used is preferably 1 to 2 moles per mole of the silyl group. The solvent is preferably tetrahydrofuran.

The reaction temperature is 0 to 30˚C, preferably 5 to 20˚C, and the reaction time is 1 to 10 hours, preferably 2 to 5 hours. In the case of purification, a preferable purification method includes a method wherein the reaction solution is poured into water and then extracted with ethyl acetate or the like followed by subjecting its concentrate to chromatographic separation or recrystallization, or alternatively the reaction solution is poured into water to form precipitates which are then filtered, washed with water and dried followed by chromatographic separation and recrystallization. In this case, the acylated cyclic tetrasaccharide, regardless of whether it is a single substance or a mixture of acylated cyclic tetrasaccharides different in the degree of substitution of the acyl group, can be used in production of the objective polyurethane.

In the cyclic tetrasaccharide, there are 12 hydroxyl groups as reactive functional groups among which 2 groups are primary hydroxyl groups and 10 groups are secondary hydroxyl groups. These two kinds of groups are generally different in reactivity, and such different reactivity can be used in selective silylation reaction of primary hydroxyl groups as described above, but as a matter of course, the hydroxyl groups subjected to silylation reaction are not limited to primary hydroxyl groups.

(b) Production of the polyurethane:

The cyclic tetrasaccharide-containing polyurethane can be produced in the following manner. That is, the acylated cyclic tetrasaccharide represented by the general formula (3) and the diol represented by the general formula (4) are reacted with the diisocyanate represented by the general formula (5) . Alternatively, the acylated cyclic tetrasaccharide represented by the general formula (3) is reacted with the diisocyanate represented by the general formula (5). In this case, a mixture of the acylated cyclic tetrasaccharide represented by the general formula (3) and the diol represented by the general formula (4) may be reacted with the diisocyanate represented by the general formula (5) (one-shot method), or the diol represented by the general formula (4) may be first reacted with the diisocyanate represented by the general formula (5) to form a prepolymer followed by reaction thereof with the acylated cyclic tetrasaccharide represented by the general formula (3) (prepolymer method 1), or the acylated cyclic tetrasaccharide represented by the general formula (3) may be first reacted with the diisocyanate represented by the general formula (5) to form a prepolymer followed by reaction thereof with the diol represented by the general formula (4) (prepolymer

method 2). In this case, the diol represented by the general formula (4) may be a single diol or a mixture of two or more different diols. When the diol is not used, the reaction can be carried out in the same manner as described above.

Examples of the diisocyanate represented by the general formula (5) used in the present invention include, diphenyl methane diisocyanate, paraphenylene diisocyanate, xylylene diisocyanate, tetramethyl xylylene diisocyanate, tolylene diisocyanate, hexamethylene diisocyanate, isophorone diisocyanate, dicyclohexyl methane diisocyanate, a prepolymer having isocyanate groups at both ends thereof, and the like. The compounds can be used singly or as a mixture of two or thereof.

The diol represented by the general formula (4) is not particularly limited insofar as it is a diol having a primary hydroxyl group. Specific examples of the diol include low-molecular-weight diols such as ethylene glycol, 1,3-propanediol, 1,4-butanediol, 1,6-hexanediol, 1,9-nonanediol, and 2,2,3,3,4,4,5,5-octafluoro-1,6-hexanediol; polyether diols such as polyethylene glycol, polytetramethylene ether glycol, polypropylene glycol, an ethylene oxide-propylene oxide copolymer, a tetrahydrofuran-ethylene oxide copolymer, and a tetrahydrofuran-propylene oxide copolymer; polyester diols such as polyethylene adipate glycol, polydiethylene adipate glycol, polypropylene adipate glycol, polybutylene adipate glycol, polyhexamethylene adipate glycol, polyneopentyl adipate glycol, and polycaprolactone glycol; polycarbonate diols such as polyhexamethylene carbonate glycol; polyolefin glycols such as polybutadiene glycol, hydrogenated polybutadiene glycol, and hydrogenated polyisoprene glycol; and high-molecular-weight diols such as bis(hydroxyethoxy-n-propyldimethylsilyl) polydimethyl siloxane. These can be used alone or as a mixture of two or more thereof.

The solvent used in producing the polyurethane may be any solvent in which the reactants and the formed polyurethane can be dissolved. Specific examples include, for example, organic solvents such as dimethyl sulfoxide (DMSO), N-methyl-2-pyrrolidone(NMP),N,N-dimethylformamide(DMF)and N,N-dimethyl acetamide (DMAc), and these solvents may be used alone or as a mixed solvent thereof.

In production of the polyurethane, a dry inert gas such as nitrogen is passed into a solution of the diisocyanate of the general formula (5), while the acylated cyclic tetrasaccharide of the general formula (3), and the diol of the general formula (4) if used, are added to the solution. The molar ratio of these compounds charged is such that the compound of the general formula (5) : the compound of the general formula (4) : the compound of the general formula (3) is preferably 3 : 0 to 2.99 : 0.01 to 3, more preferably 3 : 0.2 to 2.5 : 0.5 to 2.8. The reaction temperature is preferably 10 to 150˚C, more preferably 20 to 120˚C, and the reaction time is preferably 1 to 10 hours, more preferably 2 to 6 hours.

After the reaction is completed, the reaction solution is poured into an organic solvent such as methanol or acetone, filtered, washed, and subjected repeatedly if necessary to purification by re-precipitation to give a solid which is then dried under reduced pressure at room temperature to 100˚C for about 1 to 24 hours, whereby the polyurethane of the present invention represented by the general formula (1) can be obtained.

Then, a process for producing the cyclic tetrasaccharide-containing polyurethane represented by the general formula (2) is described. Acyl groups in the polyurethane can be easily hydrolyzed under basic conditions. The base used includes, for example, sodium alkoxide, potassium alkoxide, and the like. The solvent used includes a solvent in which the polyurethane is dissolved, for example, a single solvent such as DMF, DMAc, DMSO, toluene and xylene, and a mixture of a mixed solvent thereof in a suitable ratio with methanol. A base in an amount of 1 mol% based on the acetyl group is added to 3 to 5 wt% solution of the completely dissolved objective compound and reacted at a temperature of 0 to 50˚C for 1 to 10 hours, preferably at room temperature to a temperature of 30˚C for 2 to 7 hours, and then its filtrate is concentrated and subjected repeatedly to purification by re-precipitation with a suitable solvent such as methanol to give the objective polymer.

Now, another process for producing the cyclic tetrasaccharide-containing polyurethane represented by the general formula (2) is described. The cyclic tetrasaccharide represented by the general formula (6) and the diol represented by the general formula (4) are reacted with the diisocyanate represented by the general formula (5). Alternatively, the cyclic tetrasaccharide represented by the general formula (6) is reacted with the diisocyanate represented by the general formula (5). In this case, a mixture of the cyclic tetrasaccharide represented by the general formula (6) and the diol represented by the general formula (4) may be reacted with the diisocyanate represented by the general formula (5) (one-shot method), or alternatively the diol represented by the general formula (4) may be first reacted with the diisocyanate represented by the general formula (5) to form a prepolymer followed by reaction thereof with the cyclic tetrasaccharide represented by the general formula (6) (prepolymer method 1), or the cyclic tetrasaccharide represented by the general formula (6) may be first reacted with the diisocyanate represented by the general formula (5) to form a prepolymer followed by reaction thereof with the diol represented by the general formula (4) (prepolymer method 2). In this case, the diol represented by the general formula (4) may be a single diol or a mixture of two or more different diols. When the diol is not used, the reaction can be carried out in the same manner as described above.

The solvent in production of the polyurethane represented by the general formula (2) may be any solvent in which the reactants and the formed polyurethane can be dissolved. Specific examples of the solvent include the above-mentioned single organic solvent or mixed solvents thereof.

The reaction conditions in production of the polyurethane represented by the general formula (2) are established

such that a dry inert gas such as nitrogen is passed into a solution of the diisocyanate represented by the general formula (5), while the cyclic tetrasaccharide represented by the general formula (6), and the diol of the general formula (4) if used, are added to the solution. The molar ratio of these compounds charged is such that the compound of the general formula (5) : the compound of the general formula (4) : the compound of the general formula (6) is preferably 3 : 0 to 2.99 : 0.01 to 3, more preferably 3 : 0.2 to 2.5 : 0.5 to 2.8. The reaction temperature is preferably 10 to 150˚C, more preferably 20 to 120˚C, and the reaction time is preferably 1 to 10 hours, more preferably 2 to 6 hours.

After the reaction is completed, the reaction solution is poured into a single solvent such as methanol, acetone or water or a mixed solvent thereof to precipitate the polymer which is then filtered, washed, and subjected repeatedly if necessary to purification by re-precipitation to give a solid which is then dried under reduced pressure at room temperature to 100˚C for about 1 to 24 hours, whereby the polyurethane of the present invention represented by the general formula (2) can be obtained.

The polyurethane foam of the present invention comprises a cyclic tetrasaccharide, a polyol and a polyisocyanate. As the process for producing the same, a process for producing the polyurethane foam according to the present invention described later in detail can be preferably used.

A process for producing the polyurethane foam according to the present invention comprises reacting a polyol with a polyisocyanate in the presence of a cyclic tetrasaccharide and making a foam. The polyol used in the present invention may be a polyol other than the cyclic tetrasaccharide, and examples of such a polyol include, for example, polyether diols such as polyethylene glycol, polytetramethylene ether glycol, polypropylene glycol, an ethylene oxide-propylene oxide copolymer, a tetrahydrofuran-ethylene oxide copolymer, and a tetrahydrofuran-propylene oxide copolymer; polyester diols such as polyethylene adipate glycol, polydiethylene adipate glycol, polypropylene adipate glycol, polybutylene adipate glycol, polyhexamethylene adipate glycol, polyneopentyl adipate glycol, and polycaprolactone glycol, polycarbonate diols such as polyhexamethylene carbonate glycol; polyolefin glycols such as polybutadiene glycol, hydrogenated polybutadiene glycol, and hydrogenated polyisoprene glycol; and high-molecular-weight diols such as bis(hydroxyethoxy-n-propyldimethylsilyl) polydimethyl siloxane or low-molecular-weight diols such as ethylene glycol, 1,3-propanediol, 1,4-butanediol, 1,6-hexanediol, 1,9-nonanediol, 2,2,3,3,4,4,5,5-octafluoro-1,6-hexanediol. Further, trivalent or more polyhydric alcohols such as trimethylolpropane,trimethylolethane,glycerin hexanetriol, and pentaerythritol can also be used. The polyols may be used alone or as a mixture of two or more thereof.

The polyol is a polyol having preferably 2 to 4, more preferably 2 to 3, functional groups on average, and when the average number of functional groups is 2, the hydroxyl value of the polyol is preferably 30 to 200 mg KOH/g, more preferably 40 to 180 mg KOH/g, when the average number of functional groups is 3, the hydroxyl value is preferably 40 to 220 mg KOH/g, more preferably 45 to 200 mg KOH/g, and when the average number of functional groups is 4, the hydroxyl value is preferably 50 to 240 mg KOH/g, more preferably 60 to 220 mg KOH/g, from the viewpoint of elasticity and strength, and for example, polyethylene glycol, polypropylene glycol, or the like, can be preferably used.

Examples of the polyisocyanate used in the present invention include, for example, diphenyl methane diisocyanate, paraphenylene diisocyanate, xylylene diisocyanate, tetramethyl xylylene diisocyanate, tolylene diisocyanate, hexamethylene diisocyanate, isophorone diisocyanate, dicyclohexyl methane diisocyanate, and a prepolymer having isocyanate groups at both ends thereof. Tri- or more functional isocyanates such as polymeric diphenyl methane diisocyanate and triphenyl methane triisocyanate can also be used. These can be used alone or as a mixture of two or more thereof. Preferable among those described above are tolylene diisocyanate,diphenylmethane diisocyanate,triphenylmethane triisocyanate, or the like.

In the process for producing the polyurethane foam of the present invention, any of the one-shot method, prepolymer method and pseudo-prepolymer method may be used as a system of preparing a polyurethane chain, and a slab or mold method may be used in a system of preparing the polyurethane foam. Hereinafter, the process for producing the polyurethane foam is described specifically by reference to the one-shot method in a mold system by using the respective components. The other processes can also be easily carried out by reference to the following description.

First, the polyol component (which refers hereinafter to a polyol and a cyclic tetrasaccharide), and if necessary a catalyst, a foaming agent and a foam stabilizer are weighed out, and these materials are uniformly mixed to prepare a polyol solution. Then, a polyisocyanate is weighed out and mixed with the previous polyol solution in a reactive mold and subjected to contact catalysis to give the intended foam.

For the amount of the polyol component used, the polyol/cyclic tetrasaccharide molar ratio is preferably from 2. 95/0.05 to 1.50/1. 50, more preferably 2. 75/0. 25 to 2. 00/1.00, from the viewpoint of easy balance between the elasticity and water absorbing property of the polyurethane foam. When the cyclic tetrasaccharide is lower than this range, water absorbing property is lowered, while when the polyol is lower than this range, elasticity is lowered.

For the proportion of the polyisocyanate, the ratio of the number of moles of the isocyanate group to the number of moles of the hydroxyl group contained in the polyol and in the cyclic tetrasaccharide is preferably about 0.5 to 2.0, more preferably about 0.6 to 1.5, from the viewpoint of water-absorbing property and required rigidity.

The foaming agent may be any of various foaming agents used generally in polyurethane production. Examples

of such a foaming agent include water, an organic foaming agent and an inorganic foaming agent. The organic foaming agent includes, for example, a nitroalkane, nitrourea, an aldoxime, an active methylene compound, an acid amide, a tertiary alcohol and oxalic acid hydrate. The inorganic foaming agent includes, for example, trichloromonofluoromethane, dichlorodifluoromethane, boric acid, solid carbon dioxide, and aluminum hydroxide. Among these compounds, water is most preferable from the viewpoint of easiness and economic efficiency and can be used as a part or the whole of the foaming agent. The amount of the foaming agent is preferably in the range of 1 to 5 parts by weight based on 100 parts by weight of the used polyol component (that is, the polyol and the cyclic tetrasaccharide).

The catalyst used for regulating the reaction rate of the polyol component and the isocyanate includes catalysts used generally in polyurethane production, for example, organometallic salts such as dibutyltin dilaurate and stannous octoate and tertiary amines such as triethylene diamine, N-ethylmorpholine and pentamethyldiethylene triamine(PMDETA). Usually, the organometal salts are used in the range of 0.01 to 3 parts by weight, and the tertiary amines are used in the range of 0.01 to 3 parts by weight, based on 100 parts by weight of the used polyol component (that is, the polyol and the cyclic tetrasaccharide) . When the amount of the catalyst used is out of this range, there may arise inconveniences such as foam cracking, voids and closed cells.

As the foam stabilizer, a foam stabilizer for a slab foam and a foam stabilizer for a hot mold foam can be used. For example, L-520 and L582 (manufactured by Nippon Unicar Co., Ltd.), SH190, SH192, SF2904 and SZ1142 (manufactured by Dow Corning Toray Co., Ltd.) can be used. These foam stabilizers are used usually in the range of 0.5 to 3.0 parts by weight based on 100 parts by weight of the polyol component.

When the respective components described above are mixed and reacted to form a polyurethane foam, a mixing apparatus is used for stirring during reaction molding is an apparatus used generally in forming a polyurethane foam, such as a mechanical stirring machine, a high-pressure stirring machine or an air mixing machine.

In production of the polyurethane foam, heat necessary for the reaction is given by spontaneous reaction heat, but depending on the reactivity of the polyol component and polyisocyanate used, the reaction mixture may be heated to a temperature of about 100 to 150˚C. Before and/or after addition of the polyisocyanate, the reaction solution is stirred preferably for 10 seconds or more, preferably for 30 seconds or more, in order to uniformly disperse the cyclic tetrasaccharide in the polyol solution.

The curing time of the polyurethane foam, though varying depending on the type of the polyol component and polyisocyanate, the amount of a catalyst added for promoting the reaction, and the reaction temperature, is generally 5 minutes or more at normal temperature.

In the present invention, known additives such as a flame retardant (for example, tris(2,3-dichloropropyl) phosphate, and the like), a coloring agent, an antioxidant, a viscosity reducing agent (for example, propylene carbonate, and the like) may be used in addition to the components described above, depending on the intended use and object of the foam.

## Examples

Hereinafter, the present invention is described in more detail by reference to Examples, but the present invention is not limited thereto.

The monomer compounds used in polymerization in Examples 1 to 16, Comparative Examples 1 to 2 and Synthesis Examples are abbreviated as follows:

CTS = cyclic tetrasaccharide
CTSAc = decaacetyl cyclic tetrasaccharide
βCD = β-cyclodextrin
MDI = methane diphenyl diisocyanate
PPG = polypropylene glycol
PTMG = polytetramethylene glycol
PSiG = bis (hydroxyethoxy-n-propyldimethylsilyl) polydimethyl siloxane
OFG = 2,2,3,3,4,4,5,5-octafluoro-1,6-hexanediol

### Synthesis Example 1

### Synthesis of di (t-butyldimethylsilyl) cyclic tetrasaccharide

Because a cyclic tetrasaccharide (Hayashibara Biochemical Laboratories, Inc.) contained about 12% water, it was used after complete dehydration by vacuum drying at 100˚C for 12 hours.

Pyridine (200 ml) was introduced into a four-neck flask (flushed previously with a nitrogen gas) equipped with a mechanical stirrer and a thermometer, and 4-dimethyl aminopyridine (0.70 g) and the cyclic tetrasaccharide (30.00 g) were added

thereto and cooled to 5˚C on an ice bath. A solution of t-butyl dimethylsilyl chloride (14.68 g) in pyridine (100 ml) was added dropwise to the dispersion solution of the cyclic tetrasaccharide over 120 minutes, and the mixture was stirred at 5˚C for 1 hour and then stirred at room temperature for 3 hours. The pyridine was distilled away under reduced pressure, and water (500 ml) was introduced into the resulting concentrate which was then stirred to form precipitates. The precipitates were filtered, washed with water, and dried under vacuum to give a white solid (yield 90%).
By its proton NMR, the product was confirmed to be the objective product.
Proton NMR (δ (ppm): heavy methanol)
0.05; 18H; $(CH_3)_3CSi$-
0.82; 12H; $(CH_3)_2Si$-
2.95 to 5.50; 28H; $-CH_2$-, -CH-
4.75; 10H; -OH

Synthesis Example 2

Synthesis of di(t-butyldimethylsilyl) decaacetyl cyclic tetrasaccharide

Di(t-butyldimethylsilyl) cyclic tetrasaccharide (42.5 g), 4-dimethyl aminopyridine (0.60 g), pyridine (120 ml) and acetic anhydride (280 g) were introduced into a four-neck flask (flushed previously with a nitrogen gas) equipped with a magnetic stirrer and a thermometer, and then stirred for 20 hours at 70˚C. The pyridine in the reaction solution was removed by distillation, and the resulting residues were introduced into ice water to precipitate a product which was then washed with water, filtered and dried under vacuum to give crude crystals. The crude crystals were purified by silica gel column chromatography (developing solvent: ethyl acetate/toluene = 1/1 (ratio by volume)) to give a white solid (yield 39%).
By its proton NMR, the product was confirmed to be the objective product.
Proton NMR (solvent: heavy chloroform)
0.05; 18H; $(CH_3)_3CSi$-
0.82; 12H; $(CH_3)_2Si$-
1.90 to 2.10; 30H; $(CH_3OCO)$-
3.10 to 5.20; 28H; $-CH_2$-, -CH-

Synthesis Example 3

Synthesis of decaacetyl cyclic tetrasaccharide (CTSAc)

Di(t-butyldimethylsilyl) decaacetyl cyclic tetrasaccharide (6.0 g) and tetrahydrofuran (40 ml) were introduced into a four-neck flask (flushed previously with a nitrogen gas) equipped with a magnetic stirrer and a thermometer, and then cooled to 5˚C on an ice bath. A solution of tetrabutyl ammonium fluoride in tetrahydrofuran (1 M, 18.5 ml) was added dropwise to the solution and stirred for 1 hour, and after the ice bath was removed, the reaction mixture was stirred at room temperature for 2 hours. The reaction solution was poured into water and then extracted with ethyl acetate, and the ethyl acetate solution was washed with water, dried over sodium sulfate anhydride and concentrated. The concentrate was purified by silica gel column chromatography (developing solvent: ethyl acetate) to give a white solid (yield 40%).
By its proton NMR, the product was confirmed to be the objective product.
Proton NMR (solvent: heavy chloroform)
1.90 to 2.15; 30H; $(CH_3OCO)$-
3.45 to 5.80; 28H; $-CH_2$-, -CH-

Example 1

Synthesis of CTSAc-MDI (molar ratio 1/1) polyurethane

Decaacetyl cyclic tetrasaccharide (2.50 g), dimethyl sulfoxide (15 ml) and methane diphenyl diisocyanate (0.61 g) were introduced into a four-neck flask (flushed previously with a nitrogen gas) equipped with a mechanical stirrer, and then heated gradually from room temperature to 120˚C under stirring, and at this temperature, the mixture was reacted for 4 hours. The reaction solution was introduced into methanol to precipitate a product which was then filtered, washed with methanol and dried under vacuum to give a product (yield 85%) .
By its proton NMR, the product was confirmed to be the objective product..
Proton NMR (solvent: heavy dimethyl sulfoxide)

1.90 to 2.15; 30H; ($CH_3OCO$)-
3.45 to 5.85; 28H; -$CH_2$-, -CH-
3.76; 2H; -$CH_2$-
7.05, 7.32; 8H; -$C_6H_4$
8.52, 9.60; 2H; -NH-CO-

Example 2

Synthesis of CTSAc-PPG-MDI (molar ratio 1/2/3) polyurethane (one-shot method)

Decaacetyl cyclic tetrasaccharide (1.00 g), polypropylene glycol (average molecular weight 700, 1.37 g), dimethyl sulfoxide (15 ml) and methane diphenyl diisocyanate (0.77 g) were introduced into a four-neck flask (flushed previously with a nitrogen gas) equipped with a mechanical stirrer, and then heated gradually from room temperature to 120˚C under stirring, and at this temperature, the mixture was reacted for 4 hours. The reaction solution was introduced into methanol to precipitate a product which was then filtered, washed with methanol and dried under vacuum to give a product (yield 90%).
By its proton NMR, the product was confirmed to be the objective product.
Proton NMR (solvent: heavy dimethyl sulfoxide)
1.05, 1.20; 24H; $CH_3$-
1. 90 to 2.15; 10H; ($CH_3OCO$) -
3.20 to 3.60; 24H; -O-CH-$CH_2$-O
3.45 to 5.85; 9H; -$CH_2$-, -CH-
3.76; 2H; -$CH_2$-
7.05, 7.32; 8H; -$C_6H_4$
8.52, 9.60; 2H; -NH-CO-

Example 3

Synthesis of CTS-PPG-MDI (molar ratio 1/2/3) polyurethane (deacetylation)

The polyurethane (1.0g) obtained in Example 2 and methanol (25 ml) were introduced into a one-neck flask equipped with a magnetic stirrer, and the polyurethane was dispersed in methanol. A solution of sodium methoxide in methanol (28 wt%, 1.23 g) was added thereto and reacted at room temperature for 2 hours. Precipitates were filtered, washed with methanol and dried under vacuum to give a product (yield 100%).
By its proton NMR, the product was confirmed to be the objective product.
Proton NMR (solvent: heavy dimethyl sulfoxide)
1.05, 1.20; 24H; $CH_3$-
3.20 to 3.60; 24H; -O-CH-$CH_2$-O-
3.60 to 5.40; 9H; -$CH_2$-, -CH-
3.76; 2H; -$CH_2$-
7.05, 7.32; 8H; -$C_6H_4$
8.52, 9.60; 2H; -NH-CO-

Example 4

Synthesis of CTS-PPG-MDI (molar ratio 1/2/3) polyurethane (prepolymer method 1)

Polypropylene glycol (average molecular weight 700, 6.48 g), dimethyl acetamide (60ml) and methane diphenyl diisocyanate (3.64 g) were introduced into a four-neck flask (flushed previously with a nitrogen gas) equipped with a mechanical stirrer, and then heated gradually from room temperature to 90˚C under stirring, to react the mixture for 1 hour. Then, this reaction solution was cooled to room temperature, then cyclic tetrasaccharide (3.00 g) was added thereto, and at this temperature, the mixture was reacted for 4 hours. The reaction solution was introduced into methanol to precipitate a product which was then filtered, washed with methanol and dried under vacuum to give a product (yield 77%).
By its proton NMR, the product was confirmed to be the objective product.
Proton NMR (solvent: heavy dimethyl sulfoxide)
1.05, 1.20; 24H; $CH_3$-
3.20 to 3.60; 24H; -O-CH-$CH_2$-

3.60 to 5.40; 9H; $-CH_2-$, -CH-
3.76; 2H; $-CH_2-$
7.05, 7.32; 8H; $-C_6H_4$
8.52, 9.60; 2H; -NH-CO-

Example 5

Synthesis of CTS-PPG-MDI (molar ratio 0.5/2.5/3) polyurethane (prepolymer method 1)

Using polypropylene glycol (average molecular weight 700, 8.10 g), dimethyl acetamide (65 ml), methane diphenyl diisocyanate (4.25 g) and cyclic tetrasaccharide (3.00 g), the objective polyurethane was obtained (yield 90%) in the same manner as in Example 4.

Example 6

Synthesis of CTS-PPG-MDI (molar ratio 0.25/2.75/3) polyurethane (prepolymer method 1)

Using polypropylene glycol (average molecular weight 700, 8.91 g), dimethyl sulfoxide (60 ml), methane diphenyl diisocyanate (4.55 g) and cyclic tetrasaccharide (3.00 g), the objective polyurethane was obtained (yield 92%) in the same manner as in Example 4.

Example 7

Synthesis of CTS-PPG-MDI (molar ratio 0.1/2.9/3) polyurethane (prepolymer method 1)

Using polypropylene glycol (average molecular weight 700, 9.40 g), dimethyl acetamide (65 ml), methane diphenyl diisocyanate (4.73 g) and cyclic tetrasaccharide (3.00 g), the objective polyurethane was obtained (yield 90%) in the same manner as in Example 4.

Example 8

Synthesis of CTS-PTMG-MDI (molar ratio 1/2/3) polyurethane (prepolymer method 1)

Using polytetramethylene glycol (average molecular weight 1000, 9.26 g), dimethyl sulfoxide (65 ml), methane diphenyl diisocyanate (3.64 g) and cyclic tetrasaccharide (3.00 g), the objective polyurethane was obtained (yield 93%) in the same manner as in Example 4.

Example 9

Synthesis of CTS-PTMG-MDI (molar ratio 0.5/2.5/3) polyurethane (prepolymer method 1)

Using polytetramethylene glycol (average molecular weight 1000, 11.57 g), dimethyl acetamide (65 ml), methane diphenyl diisocyanate (4.25 g) and cyclic tetrasaccharide (3. 00 g), the objective polyurethane was obtained (yield 92%) in the same manner as in Example 4.

Example 10

Synthesis of CTS-PTMG-MDI (molar ratio 0.1/2.9/3) polyurethane (prepolymer method 1)

Using polytetramethylene glycol (average molecular weight 1000, 13.4 g), dimethyl sulfoxide (65 ml), methane diphenyl diisocyanate (4.73 g) and cyclic tetrasaccharide (3.00 g), the objective polyurethane was obtained (yield 90%) in the same manner as in Example 4.

Example 11

Synthesis of CTS-PSiG-PPG-MDI (molar ratio 1/0.25/1.75/3) polyurethane (prepolymer method 1)

Using polypropylene glycol (average molecular weight 700, 5.67 g), dimethyl acetamide (65 ml), methane

diphenyl diisocyanate (3. 64 g), cyclic tetrasaccharide (3. 00 g) and PSiG (average molecular weight 1000, 1.16 g), the objective polyurethane was obtained (yield 90%) in the same manner as in Example 4.

Example 12

Synthesis of CTS-PSiG-PPG-MDI (molar ratio 1/0.1/1.9/3) polyurethane (prepolymer method 1)

Using polypropylene glycol (average molecular weight 700, 6.16 g), dimethyl sulfoxide (65 ml), methane diphenyl diisocyanate (3. 64 g), cyclic tetrasaccharide (3.00 g) and PSiG (average molecular weight 1000, 0.46 g), the objective polyurethane was obtained (yield 91%) in the same manner as in Example 4.

Example 13

Synthesis of CTS-PSiG-PPG-MDI (molar ratio 0.5/0.1/2.4/3) polyurethane (prepolymer method 1)

Using polypropylene glycol (average molecular weight 700, 7.78 g), dimethyl acetamide (65 ml), methane diphenyl diisocyanate (4.25 g), cyclic tetrasaccharide (3.00 g) and PSiG (average molecular weight 1000, 0.46 g), the objective polyurethane was obtained (yield 93%) in the same manner as in Example 4.

Example 14

Synthesis of CTS-PSiG-PPG-MDI (molar ratio 0.5/0.2/2.3/3) polyurethane (prepolymer method 1)

Using polypropylene glycol (average molecular weight 700, 7.45 g), dimethyl sulfoxide (65 ml), methane diphenyl diisocyanate (4.25 g), cyclic tetrasaccharide (3.00 g) and PSiG (average molecular weight 1000, 0.93 g), the objective polyurethane was obtained (yield 91%) in the same manner as in Example 4.

Example 15

Synthesis of CTS-OFG-PPG-MDI (molar ratio 0.5/0.1/2.4/3) polyurethane (prepolymer method 1)

Using polypropylene glycol (average molecular weight 700, 10.37 g), dimethyl acetamide (70 ml), methane diphenyl diisocyanate (4.63 g), cyclic tetrasaccharide (2. 00 g) and OFG (0. 16 g), the objective polyurethane was obtained (yield 90%) in the same manner as in Example 4.

Example 16

Synthesis of CTS-OFG-PPG-MDI (molar ratio 0.5/0.2/2.3/3) polyurethane (prepolymer method 1)

Using polypropylene glycol (average molecular weight 700, 9.94 g), dimethyl sulfoxide (70 ml), methane diphenyl diisocyanate (4.63 g), cyclic tetrasaccharide (2.00 g) and OFG (0.32 g), the objective polyurethane was obtained (yield 88%) in the same manner as in Example 4.

Comparative Example 1

Synthesis of PPG-MDI (molar ratio 1/1) polyurethane

Polypropylene glycol (average molecular weight 700, 14.70 g), dimethyl acetamide (100 ml) and methane diphenyl diisocyanate (5.50 g) were introduced into a four-neck flask (flushed previously with a nitrogen gas) equipped with a mechanical stirrer, and then heated gradually from room temperature to 120˚C under stirring, and at this temperature, the mixture was reacted for 4 hours. The reaction solution was introduced into methanol to precipitate a product which was then filtered, washed with methanol and dried under vacuum to give a product (yield 90%).
By its proton NMR, the product was confirmed to be the objective product.
Proton NMR (solvent: heavy dimethyl sulfoxide)
1.05, 1.20; 42H; $-CH_3-$
3.20 to 3.60; 42H; $-O-CH-CH_2-O-$
3.76; 2H; $-CH_2-$
7.05, 7.32; 8H; $-C_6H_4$

8.52; 2H; -NH-CO-

Comparative Example 2

Synthesis of βCD-PPG-MDI (molar ratio 1/2/3) polyurethane (prepolymer method 1)

Polypropylene glycol (average molecular weight 700, 6.48 g), dimethyl acetamide (60ml) and methane diphenyl diisocyanate (3.64 g) were introduced into a four-neck flask (flushed previously with a nitrogen gas) equipped with a mechanical stirrer, and then heated gradually from room temperature to 90˚C under stirring. Then, β-cyclodextrin (5.00 g) was added thereto and then heated gradually to 120˚C, and at this temperature, the mixture was reacted for 4 hours. The reaction solution was introduced into methanol to precipitate a product which was then filtered, washed with methanol and dried under vacuum to give a product (yield 77%).
By its proton NMR, the product was confirmed to be the objective product.
Proton NMR (solvent: heavy dimethyl sulfoxide)
1.05, 1.20; 24H; $CH_3$-
3.0 to 3.60; 24H; -O-CH-$CH_2$-O-
3.40 to 5.80; 17H; -$CH_2$-, -CH-
3.76; 2H; -$CH_2$-
7.05, 7.32; 8H; -$C_6H_4$
8.52; 2H; -NH-CO-

Comparative Example 3

Synthesis of sucrose-PPG-MDI (molar ratio 1/2/3) polyurethane (prepolymer method 1)

Polypropylene glycol (average molecular weight 700, 6.48 g), dimethyl acetamide (60ml) and methane diphenyl diisocyanate (3.64 g) were introduced into a four-neck flask (flushed previously with a nitrogen gas) equipped with a mechanical stirrer, and then heated gradually from room temperature to 90˚C under stirring. Upon adding sucrose (1.50 g) thereto, the reaction solution formed a jelly. The reaction solution was heated gradually to a temperature of 120˚C, and at this temperature, the reaction solution was reacted for 4 hours. The reaction solution was introduced into methanol to precipitate a product which was then filtered, washed with methanol and dried under vacuum to give a product (yield 77%).
Because this product was not dissolved in any solvent, its proton NMR could not be measured.

<Preparation of a pressed film>

Each of the various polyurethanes obtained in Examples 1 to 16 and Comparative Examples 1 and 2 was used to prepare a pressed film with a pressure molding machine. Thepreparation conditions were as follows: the temperature was 150 to 160˚C, the pressure was 3 MPa, and the time was 2 minutes. From the polyurethane in Example 1, a film could not be obtained.

Evaluation Methods

Molecular weight: The weight-average molecular weight (Mw) of the acetylated cyclic tetrasaccharide-containing polyurethane produced in each of Examples 1 to 16 and Comparative Examples 1 and 2 was determined by gel permeation chromatography (GPC) on a polystyrene column using standard polystyrene (PSt) as the standard with chloroform as the developing solvent. The weight-average molecular weight of the cyclic tetrasaccharide-containing polyurethane, on the other hand, was determined using standard polyethylene (PE) as the standard with DMF (dimethylformamide) as the developing solvent.
Softening point (˚C): The softening point of the polyurethane produced in each of Examples 1 to 16 and Comparative Examples 1 and 2 was determined by measuring the temperature at which the sample was melted by heating (5˚C/min.) from room temperature with a melting-point apparatus.
Water absorption: The film (weight $W_0$) prepared above was dipped in purified water at 20˚C and then measured for its weight ($W_t$) at regular time intervals, and from its increase in weight, the water absorption was determined according to the following equation:

$$\text{Water absorption (\%)} = (W_t - W_0)\ 100/W_0$$

The results are shown in Tables 1-1 to 1-3.

Table 1-1

| | | Example 1 | Example 2 | Example 3 | Example 4 | Example 5 | Example 6 |
|---|---|---|---|---|---|---|---|
| Charging molar ratio | MDI | 1 | 3 | 3 | 3 | 3 | 3 |
| | PPG | - | 2 | 2 | 2 | 2.5 | 2.75 |
| | PTMG | - | - | - | - | - | - |
| | SiG | - | - | - | - | - | - |
| | OFG | - | - | - | - | - | - |
| | CTSAc | 1 | 1 | 1 | - | - | - |
| | CTS | - | - | - | 1 | 0.5 | 0.25 |
| | βCD | - | - | - | - | - | - |
| | Hydrolysis | - | - | ○ | - | - | - |
| Mw(RI) | | 26,300 | 22,900 | 43,000 | 32,000 | 20,000 | 26,000 |
| Softening point (˚C) | | 210 | 110 | 175 | 185 | 170 | 170 |
| Water absorption (%) | after 0.5 hour | - | 5 | 11 | 26 | 26 | 20 |
| | after 1 hour | - | 6 | 18 | 37 | 28 | 25 |
| | after 3 hours | - | 8 | 29 | 47 | 36 | 30 |

Table 1-2

| | | Example 7 | Example 8 | Example 9 | Example 10 | Example 11 | Example 12 |
|---|---|---|---|---|---|---|---|
| Charging molar ratio | MDI | 3 | 3 | 3 | 3 | 3 | 3 |
| | PPG | 2.9 | - | - | - | 1.75 | 1.9 |
| | PTMG | - | 2 | 2.5 | 2.9 | - | - |
| | SiG | - | - | - | - | 0.25 | 0.1 |
| | OFG | - | - | - | - | - | - |
| | CTSAc | - | - | - | - | - | - |
| | CTS | 0.1 | 1 | 0.5 | 0.1 | 1 | 1 |
| | βCD | - | - | - | - | - | - |
| | Hydrolysis | - | - | - | - | - | - |
| Mw(RI) | | 24,000 | 32,000 | 64,000 | 62,000 | 32,000 | 41,000 |
| Softening point (˚C) | | 170 | 175 | 175 | 180 | 185 | 190 |
| Water absorption (%) | after 0.5 hour | 27 | 24 | 14 | 10 | 10 | 24 |
| | after 1 hour | 29 | 27 | 17 | 12 | 14 | 24 |
| | after 3 hours | 32 | 35 | 24 | 14 | 14 | 28 |

Table 1-3

| | | Example 13 | Example 14 | Example 15 | Example 16 | Comparative Example 1 | Comparative Example 2 |
|---|---|---|---|---|---|---|---|
| Charging molar ratio | MDI | 3 | 3 | 3 | 3 | 1 | 3 |
| | PPG | 2.4 | 2.3 | 2.4 | 2.3 | 1 | 2 |
| | PTMG | - | - | - | - | - | - |
| | SiG | 0.1 | 0.2 | - | - | - | - |
| | OFG | - | - | 0.1 | 0.2 | - | - |
| | CTSAc | - | - | - | - | - | - |
| | CTS | 0.5 | 0.5 | 0.5 | 0.5 | - | - |
| | βCD | - | - | - | - | - | 1 |
| | Hydrolysis | - | - | - | - | - | - |
| Mw(RI) | | 20,000 | 20,000 | 41,000 | 97,000 | 164,000 | 89,000 |
| Softening point (˚C) | | 180 | 170 | 185 | 180 | 179 | 235 |
| Water absorption (%) | after 0.5 hour | 24 | 10 | 28 | 28 | 4 | 8 |
| | after 1 hour | 24 | 15 | 28 | 28 | 4 | 13 |
| | after 3 hours | 28 | 15 | 28 | 28 | 4 | 15 |

From the above results, it was revealed that the polyurethanes obtained in Examples 1 to 16 in the present invention are thermoplastic, and the polyurethanes in Examples 2 to 16 are excellent in film formability, and the films (films of non-acylated CTS-containing polyurethane) in Examples 3 to 16 have water absorption equal to or higher than that of the polyurethanes in Comparative Examples 1 and 2.

Examples A1 to A16 and Comparative Examples B1 to B8

Cyclic tetrasaccharide, trehalose and cyclodextrin were dried with a vacuum dryer, and after drying, their moisture contents were determined with a Karl-Fischer moisture titrator (MKC-610DT manufactured by Kyoto Electronics Manufacturing Co., Ltd.) (moisture contents are shown in the table). Polyol was dried with a molecular sieve, and its moisture content determined with the moisture titrator was 0%.

Using each of the formulations shown in Tables 2-1 to 2-4, a polyurethane foam was produced by free foaming in a hand mixing method. That is, a foam stabilizer, a catalyst, and water were mixed in advance in a predetermined molar amount or part ratio with polypropylene glycol, polypropylene glycol trioltype,polytetramethyleneglycol,cyclic tetrasaccharide, trehalose or cyclodextrin (the polyol component in the table refers to CTS, β-cyclodextrin, trehalose, polypropylene glycol, polypropylene glycol triol type and polytetramethylene glycol), and a catalyst was added to, and mixed with, the mixture followed by rapidly adding diisocyanate and mixing them with a mixer at 3000 rpm for 30 to 50 seconds. The resulting mixture was poured into a 15 cm×10 cmx3 cm container having an open upper part, then foamed and cured at room temperature for 2 minutes and then at 50˚C for 3 hours to give a polyurethane foam.

Then, the porosity, density and water absorption thereof were measured by the following methods. The results are shown in Tables 2-1 to 2-4.

[Methods of measuring porosity and density]

(1) The prepared sample is cut into cubes about 2 cm on a side, and each side is measured to accurately determine

its volume (V $cm^3$). The sample weight (W g) is also determined.
(2) The density ($g/cm^3$) is determined according to the following equation:

$$\text{Density } (g/cm^3) = W/V$$

(3) A multi-picnometer (manufactured by Quantachrome Instrument) is used to determine the closed cell volume (Vcc), and the porosity (%) is determined according to the following equation:

$$\text{Porosity } (\%) = (V - Vcc) \times 100/V$$

[Method of measuring water absorption]

(1) The prepared sample is cut in a form of 5 cm (length)x5 cm (width)×2 cm (thickness).
(2) The weight ($W_0$) of the cut sample is accurately measured.
(3) A sufficient amount of water adjusted to 20˚C is placed in a vat, and the sample in (2) is gently floated on the surface of water.
(4) After 30 minutes, the sample is gently removed and then left for 5 minutes on a stainless-steel mesh with an opening of 5 mm, to remove water of adhesion.
(5) The weight ($W_1$) of the sample having water absorbed therein in (4) is accurately measured.
(6) The water absorption (M) per g is determined according to the following equation:

$$M\ (\%) = (W_1 - W_0) \times 100/W_0$$

(7) 60 minutes thereafter, the water absorption is determined in the same manner, and when saturation is reached, the water absorption is determined as saturation water absorption (%).

In Tables 2-1 to 2-4, the compounds used in polymerization are abbreviated as follows:

CTS = cyclic tetrasaccharide
βCD = β-cyclodextrin
TRH = trehalose
MDI = methane diphenyl diisocyanate
TDI = tolylene diisocyanate
PPG 700 = polypropylene glycol (molecular weight 700, hydroxyl value 160 mg KOH/g)
PPG 2000 =polypropylene glycol (molecular weight 2000, hydroxyl value 56 mg KOH/g)
PPG 700T = polypropylene glycol triol type (molecular weight 700, hydroxyl value 240 mg KOH/g)
PTMG 1000 = polytetramethylene glycol (molecular weight 1000, hydroxyl value 112 mg KOH/g)
SH190, SH192, SF2904 and SZ1142 = foam stabilizers (manufactured by Dow Corning Toray Co., Ltd.)
DBTDL = dibutyltin dilaurate

Table 2-1

| Examples | | | A1 | A2 | A3 | A4 | A5 | A6 |
|---|---|---|---|---|---|---|---|---|
| Charging molar ratio | MDI | | 3.85 | 3.85 | 3.85 | 3.85 | 3.85 | 3.85 |
| | TDI | | - | - | - | - | - | - |
| | PPG700 | | 2 | 2 | 2 | 2 | 2 | 2 |
| | PPG700T | | - | - | - | - | - | - |
| | PPG2000 | | - | - | - | - | - | - |
| | PTMG1000 | | - | - | - | - | - | - |
| | CTS | Molar ratio | 1 | 1 | 1 | 1 | 1 | 1 |
| | | Moisture content (%) | 5 | 5 | 5 | 5 | 5 | 5 |
| | βCD | Molar ratio - | | - | - | - | - | - |
| | | Moisture content (%) | - | - | - | - | - | - |
| | TRH | Molar ratio - | | - | - | - | - | - |
| | | Moisture content (%) | - | - | - | - | - | - |
| Weight ratio (%) to the polyol component | Foam stabilizer | SH190 | - | - | 1 | - | - | - |
| | | SH192 | - | - | - | 1 | - | - |
| | | SF2904 | - | - | - | - | 1 | - |
| | | SZ1142 | - | - | - | - | - | 1 |
| | Catalyst | DBTDL | 2 | 2 | 2 | 2 | 2 | 2 |
| | Water | Saccharide contained | 1.6 | 1.6 | 1.6 | 1.6 | 1.6 | 1.6 |
| | | Added | 1.0 | 0.7 | 0 | 0 | 0 | 0 |
| | | Total | 2.6 | 2.3 | 1.6 | 1.6 | 1.6 | 1.6 |
| Physical properties of foam | Porosity (%) | | 82 | 83 | 95 | 82 | 79 | 81 |
| | Density (g/cm$^3$) | | 0.26 | 0.18 | 0.17 | 0.167 | 0.16 | 0.178 |
| | Saturation (%) water absorption | | 263 | 355 | 405 | 413 | 435 | 376 |

Table 2-2

| Examples | | | A7 | A8 | A9 | A10 | A11 | A12 |
|---|---|---|---|---|---|---|---|---|
| Charging molar ratio | MDI | | 3.96 | - | - | - | - | - |
| | TDI | | - | 3.85 | 3.85 | 3.85 | 3.85 | 3.85 |
| | PPG700 | | - | 2 | 2 | 2 | 2 | 2 |
| | PPG700T | | - | - | - | - | - | - |
| | PPG2000 | | 2 | - | - | - | - | - |
| | PTMG1000 | | - | - | - | - | - | - |
| | CTS | Molar ratio | 1 | 1 | 1 | 1 | 1 | 1 |
| | | Moisture content (%) | 3 | 5 | 5 | 12 | 5 | 5 |
| | βCD | Molar ratio - | | - | - | - | - | - |
| | | Moisture content (%) | - | - | - | - | - | - |
| | TRH | Molar ratio - | | - | - | - | - | - |
| | | Moisture content (%) | - | - | - | - | - | - |
| Weight ratio (%) to the polyol component | Foam stabilizer | SH190 | 1 | - | - | - | 1 | - |
| | | SH192 | - | - | - | - | - | 1 |
| | | SF2904 | - | - | - | - | - | - |
| | | SZ1142 | - | - | - | - | - | - |
| | Catalyst | DBTDL | 2 | 2 | 2 | 2 | 2 | 2 |
| | Water | Saccharide contained | 3.0 | 1.6 | 1.6 | 3.8 | 1.6 | 1.6 |
| | | Added | 0 | 0.7 | 0 | 0.7 | 0 | 0 |
| | | Total | 3.0 | 2.3 | 1.6 | 4.5 | 1.6 | 1.6 |
| Physical properties of foam | Porosity (%) | | 79 | 93 | 92 | 88 | 93 | 94 |
| | Density ($g/cm^3$) | | 0.27 | 0.07 | 0.10 | 0.10 | 0.074 | 0.065 |
| | Saturation (%) water absorption | | 233 | 993 | 604 | 501 | 970 | 1180 |

Table 2-3

| Examples | | | A13 | A14 | A15 | A16 |
|---|---|---|---|---|---|---|
| Charging molar ratio | MDI | | - | - | - | - |
| | TDI | | 3.85 | 3.85 | 3.85 | 3.85 |
| | PPG700 | | 2 | 2 | - | - |
| | PPG700T | | - | - | 2 | - |
| | PPG2000 | | - | - | - | - |
| | PTMG1000 | | - | - | - | 2 |
| | CTS | Molar ratio | 1 | 1 | 1 | 1 |
| | | Moisture content (%) | 5 | 5 | 5 | 5 |
| | βCD | Molar ratio - | | - | - | - |
| | | Moisture content (%) | - | - | - | - |
| | TRH | Molar ratio - | | - | - | - |
| | | Moisture content (%) | - | - | - | - |
| Weight ratio (%) to the polyol component | Foam stabilizer | SH190 | - | - | - | 1 |
| | | SH192 | - | - | 1 | - |
| | | SF2904 | 1 | - | - | - |
| | | SZ1142 | - | 1 | - | - |
| | Catalyst | DBTDL | 2 | 2 | 2 | 2 |
| | Water | Saccharide contained | 1.6 | 1.6 | 1.6 | 1.2 |
| | | Added | 0 | 0 | 0 | 0.7 |
| | | Total | 1.6 | 1.6 | 1.6 | 1.9 |
| Physical properties of foam | Porosity (%) | | 88 | 81 | 88 | 78 |
| | Density ($g/cm^3$) | | 0.075 | 0.079 | 0.14 | 0.17 |
| | Saturation (%) water absorption | | 1000 | 835 | 495 | 238 |

Table 2-4

| Comparative Examples | | | B1 | B2 | B3 | B4 | B5 | B6 | B7 | B8 |
|---|---|---|---|---|---|---|---|---|---|---|
| Charging molar ratio | MDI | | 4.08 | 3.85 | - | - | - | - | - | - |
| | TDI | | - | - | 3.85 | 3.85 | 3.85 | 3.85 | 3.85 | 3.85 |
| | PPG700 | | 2 | 2 | 2 | 3 | 3 | - | 2 | 2 |
| | PPG700T | | - | - | - | - | - | 3 | - | - |
| | PPG2000 | | - | - | - | - | - | - | - | - |
| | PTMG1000 | | - | - | - | - | - | - | - | - |
| | CTS | Molar ratio - | | - | - | - | - | - | 1 | 1 |
| | | Moisture content (%) | - | - | - | - | - | - | 5 | 5 |
| | βCD | Molar ratio | 1 | - | - | - | - | - | - | - |
| | | Moisture content (%) | 5 | - | - | - | - | - | - | - |
| | TRH | Molar ratio - | | 1 | 1 | - | - | - | - | - |
| | | Moisture content (%) | - | 5 | 5 | - | - | - | - | - |
| Weight ratio (%) to the polyol component | Foam stabilizer | SH190 | - | - | - | - | 1 | 1 | - | - |
| | | SH192 | - | - | - | - | - | - | 1 | 1 |
| | | SF2904 | - | - | - | - | - | - | - | - |
| | | SZ1142 | - | - | - | - | - | - | - | - |
| | Catalyst | DBTDL | 2 | 2 | 2 | 2 | 2 | 2 | 2 | 2 |
| | Water | Saccharide contained | 2.2 | 1.0 | 1.0 | - | - | - | 1.6 | 1.6 |
| | | Added | 1.0 | 0.7 | 0.7 | 0.7 | 0.7 | 0.7 | 3.9 | 5.4 |
| | | Total | 3.2 | 1.7 | 1.7 | 0.7 | 0.7 | 0.7 | 5.5 | 7.0 |
| Physical properties of foam | Porosity (%) | | 70 | 64 | 51 | Foam cannot be measured due to its viscosity. | | 80 | Foam cannot be measured due to its brittleness. | |
| | Density ($g/cm^3$) | | 0.44 | 0.37 | 0.36 | | | 0.20 | | |
| | Saturation (%) water absorption | | 80 | 70 | 45 | | | 325 | | |

The results revealed that for example when Examples A1 and A8 are compared with Comparative Examples B1 to B3, Examples A1 and A8 have water absorption that is at least 3-times higher than where the same molar amount of β-cyclodextrin is used (Comparative Example B1) or where the same molar amount of trehalose is used (Comparative Examples B2 and B3), and the polyurethane foams in Examples A1 and A8 are those of lower density having high porosity equal to or higher than, and high water absorption performance equal to or higher than, those of the P-cyclodextrin- or trehalose-containing polyurethane foams in Comparative Examples B1 to B3, and thus the effectiveness of the present invention in conferring water-absorbing property is thus confirmed. Any products in the Examples other than Examples A1 and A8 show high water absorption percentages equal to or higher than those of the conventional products, and it was confirmed that particularly when TDI is used as polyisocyanate, a very high water absorption percentage can be obtained. The comparison between Example A15 and Comparative Example B6 revealed that water-absorbing

property is increased by replacing a part of the polyol component by cyclic tetrasaccharide.

## Claims

1. An acylated cyclic tetrasaccharide-containing polyurethane represented by the following general formula (1) :

[Formula 1]

$$-\{[CO-NH-R^1-NH-CO]-[O-R^2-O]\}m-\#$$

$$\#-\{[CO-NH-R^1-NH-CO]-[O-\underset{\underset{(OH)_{10-p}}{|}}{\overset{\overset{(OR^3)_p}{|}}{C}}TS-O]\}n- \quad . \qquad [1]$$

wherein $R^1$ represents a divalent aliphatic hydrocarbon group having 4 to 16 carbon atoms, a divalent aromatic hydrocarbon group having 6 to 16 carbon atoms, or a divalent aromatic substituted hydrocarbon group having 7 to 16 carbon atoms, $R^2$ represents a divalent organic group containing 1 to 100 units in total of an oxyalkylene group having 2 to 12 carbon atoms and/or an alkylene group having 2 to 6 carbon atoms and when there are a plurality of oxyalkylene groups and a plurality of alkylene groups, they may be the same or different, respectively, $R^3$ represents an acyl group having 2 to 8 carbon atoms, CTS represents a skeleton of a cyclic tetrasaccharide that is cyclo{→6)-α-D-glucopyranosyl-(1→3)-α-D-glucopyranosyl-(1 →6)-α-D-glucopyranosyl-(1→3)-α-D-glucopyranosyl-(1→}, represented by the following formula:

[Chemical formula 1]

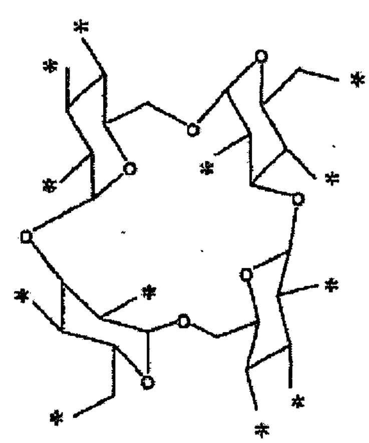

(wherein * represents the binding position of a hydroxyl group), p represents an integer of 1 to 10, m and n each represent the number of repeating units, m is an integer of 0 to 1000, n is an integer of 1 to 1000, and n/ (m + n) is a number in the range of 0.01 to 1; when there are a plurality of $R^1$s, $R^2$s or $R^3$s, each of $R^1$s, $R^2$s or $R^3$s may be the same or different, and when there are a plurality of CTS, the positions of CTS to which $R^3$ is introduced may be the same or different.

2. The acylated cyclic tetrasaccharide-containing polyurethane according to claim 1, wherein $R^3$ is an acetyl group.

3. A cyclic tetrasaccharide-containing polyurethane represented by the following general formula (2):

[Formula 2]

$$-\{[CO-NH-R^{1}-NH-CO]-[O-R^{2}-O]\}m-\#$$

$$\#-\{[CO-NH-R^{1}-NH-CO]-[O-\underset{\substack{|\\(OH)_{10}}}{CTS}-O]\}n- \qquad [2]$$

wherein $R^1$ represents a divalent aliphatic hydrocarbon group having 4 to 16 carbon atoms, a divalent aromatic hydrocarbon group having 6 to 16 carbon atoms, or a divalent aromatic substituted hydrocarbon group having 7 to 16 carbon atoms, $R^2$ represents a divalent organic group containing 1 to 100 units in total of an oxyalkylene group having 2 to 12 carbon atoms and/or an alkylene group having 2 to 6 carbon atoms and when there are a plurality of oxyalkylene groups and a plurality of alkylene groups, they may be the same or different, respectively, CTS has the same meaning as defined above, m and n each represent the number of repeating units, m is an integer of 0 to 1000, n is an integer of 1 to 1000, and n/(m + n) is a number in the range of 0.01 to 1; and when there are a plurality of $R^1$s or $R^2$s, each of $R^1$s or $R^2$s may be the same or different.

**4.** A process for producing an acylated cyclic tetrasaccharide-containing polyurethane represented by the general formula (1), comprising
reacting an acylated cyclic tetrasaccharide represented by the following general formula (3):

[Formula 3]

$$HO-\overset{\substack{(OR^{3})_{p}\\|}}{\underset{\substack{|\\(OH)_{10-p}}}{CTS}}-OH \qquad [3]$$

wherein $R^3$ represents an acyl group having 2 to 8 carbon atoms, p represents an integer of 1 to 10, CTS has the same meaning as defined above, and when there are a plurality of $R^3$s, $R^3$s may be the same or different, and
a diol represented by the following general formula (4) :

[Formula 4] $HO-R^2-OH$ [4]

wherein $R^2$ represents a divalent organic group containing 1 to 100 units in total of an oxyalkylene group having 2 to 12 carbon atoms and/or an alkylene group having 2 to 6 carbon atoms and when there are a plurality of oxyalkylene groups and a plurality of alkylene groups, they may be the same or different, respectively, with
a diisocyanate represented by the following general formula (5):

[Formula 5] $O=C=N-R^1-N=C=O$ [5]

wherein $R^1$ represents a divalent aliphatic hydrocarbon group having 4 to 16 carbon atoms, a divalent aromatic hydrocarbon group having 6 to 16 carbon atoms, or a divalent aromatic substituted hydrocarbon group having 7 to 16 carbon atoms.

**5.** A process for producing the cyclic tetrasaccharide-containing polyurethane of the general formula (2) according to claim 3, comprising
hydrolyzing an acyl-group moiety of the acylated cyclic tetrasaccharide represented by the general formula (1).

**6.** A process for producing the cyclic tetrasaccharide-containing polyurethane of the general formula (2) according to claim 3, comprising
reacting a cyclic tetrasaccharide represented by the following general formula (6):

[Formula 6]

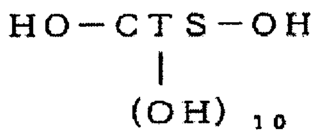

[6]

wherein CTS has the same meaning as defined above, and a diol represented by the general formula (4), with a diisocyanate represented by the general formula (5).

**7.** A polyurethane foam comprising
a cyclic tetrasaccharide that is cyclo{→6)-α-D-glucopyranosyl-(1→3)-α-D-glucopyranosyl-(1 →6)-α-D-glucopyranosyl-(1→3) -α-D-glucopyranosyl- (1→}, a polyol other than the cyclic tetrasaccharide, and a polyisocyanate.

**8.** A process for producing a polyurethane foamhaving a cyclic tetrasaccharide, comprising
reacting a polyol other than a cyclic tetrasaccharide with a polyisocyanate in the presence of a cyclic tetrasaccharide that is cyclo{→6)-α-D-glucopyranosyl-(1→3)-α-D-glucopyranosyl-(1 →6) -α-D-glucopyranosyl- (1→3) -α-D-glucopyranosyl- (1→}, and simultaneously making a foam by using, as a part or the whole of a foaming agent, water in an amount of 1 to 5 parts by weight based on 100 parts by weight of the polyol and the cyclic tetrasaccharide in total.

**9.** The process according to claim 8, wherein the polyol has 2 to 4 functional groups on average and a hydroxyl value of 30 to 240 mg KOH/g.

**10.** The process according to claim 8 or 9, wherein the polyisocyanate is tolylene diisocyanate.

**INTERNATIONAL SEARCH REPORT**

International application No.
PCT/JP2006/319417

A. CLASSIFICATION OF SUBJECT MATTER
*C08G18/32*(2006.01)i, *C07H3/06*(2006.01)n

According to International Patent Classification (IPC) or to both national classification and IPC

B. FIELDS SEARCHED

Minimum documentation searched (classification system followed by classification symbols)
C08G18/32, C07H3/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2003-160595 A (Kabushiki Kaisha Hayashibara Seibutsu Kagaku Kenkyusho), 03 June, 2003 (03.06.03), & WO 03/044032 A1 & EP 1460081 A1 & KR 2004055730 A & US 2004254367 A1 & TW 200300417 A | 1-10 |
| A | JP 2003-235596 A (Kabushiki Kaisha Hayashibara Seibutsu Kagaku Kenkyusho), 26 August, 2003 (26.08.03), (Family: none) | 1-10 |
| A | JP 5-43649 A (Sony Corp.), 23 February, 1993 (23.02.93), (Family: none) | 1-10 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 October, 2006 (23.10.06) | 31 October, 2006 (31.10.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2006/319417

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-316740 A (Canon Inc.), 02 December, 1998 (02.12.98), (Family: none) | 1-10 |
| A | JP 11-71391 A (NOF Corp.), 16 March, 1999 (16.03.99), (Family: none) | 1-10 |
| A | JP 9-12588 A (Hyoe HATAYAMA), 14 January, 1997 (14.01.97), (Family: none) | 1-10 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5086103 A
- JP 5186556 A
- JP 7053658 A
- JP 9012588 A
- JP 9104737 A
- JP 11071391 A
- JP 2003160595 A